# DEMANDE DE BREVET EUROPEEN

(11) **EP 1 384 524 A2**
(43) Date de publication de la demande: **28.01.2004**
(21) Numéro de dépôt: 03291649.6
(22) Date de dépôt: 03.07.2003
(51) Int. Cl.: B05D 5/00, C30B 29/14, A61L 27/32

(54) **Procédé permettant de recouvrir à basse température des surfaces par des phosphates apatitiques nanocristallins à partir d'une suspension aqueuse de phosphate amorphe**

(30) Priorité: 26.07.2002 FR 0209514
(71) Demandeur: Kasios, 31140 Launaguet (FR)
(72) Inventeur: Calzalbou, Sophie, 31100 Toulouse (FR); Combes, Christèle, 31520 Ramonville-Saint-Agne (FR); Rey, Christian, 31320 Aureville (FR)
(74) Mandataire: Faber, Jean-Paul

(57) **Abrégé**

L'invention concerne un procédé permettant de recouvrir une surface éventuellement poreuse, d'un substrat -notamment d'implants chirurgicaux-, par une couche de phosphate de calcium apatitique nanocristallin. Un procédé selon l'invention est mis en oeuvre, à basse température (0 à 100°C), et se caractérise en ce que la germination et la croissance cristallines de la phase apatitique s'effectuent à partir d'une suspension aqueuse de phosphate amorphe. Pour ce faire, on prépare, tout d'abord, une suspension aqueuse fraiche de phosphate amorphe. On applique cette suspension sur la surface du substrat à revêtir, et ce, par différents moyens tels que la pulvérisation, l'immersion dans la suspension (dip-coating), ou simplement à l'aide d'une brosse. On initie et éventuellement on contrôle la germination et la croissance cristalline du phosphate apatitique sur le substrat. effectuer l'hydrolyse de la phase amorphe en fluorapatite. Le spectre Infrarouge du dépôt révèle la formation d'une apatite ne contenant pas d'ions hydroxyde (figure 4).

La portée de la présente invention ne se limite pas aux détails des formes de réalisation ci-dessus considérées à titre d'exemples, mais s'étend au contraire aux modifications à la portée de l'homme de l'art.

## Description

L'invention concerne un procédé permettant de recouvrir des implants et prothèses orthopédiques par des phosphates apatitiques nanocristallins.

L'utilisation d'implants et prothèses orthopédiques est très développée, mais le problème essentiel est la jonction avec le tissu osseux vivant. Les prothèses fixées avec des ciments polymères ont des durées de vie limitées et la jonction tissu-polymère est de mauvaise qualité. Les prothèses revêtues d'hydroxyapatite permettent d'améliorer considérablement la qualité de l'interface. En effet, tous les matériaux d'implantation orthopédique biocompatibles ont montré un développement à leur surface d'une couche néoformée de cristaux d'apatite carbonatée. Les cristaux formés sont très proches de ceux du minéral osseux et semblent agir comme un leurre pour l'organisme. Ils permettent la fixation de protéines, l'adhésion et la prolifération des ostéoblastes et ainsi, la formation d'os nouveau.

La précipitation de cristaux d'os néoformés peut être accélérée par la libération de phosphate de calcium ce qui peut entraîner une augmentation locale de la sursaturation dans les fluides biologiques aux alentours de la surface de l'implant. Le développement d'une phase relativement soluble de phosphate de calcium ou de carbonate de calcium à la surface de l'implant devrait favoriser la formation d'une couche d'apatite carbonatée. Par conséquent, la présence d'une phase mal cristallisée de phosphate de calcium ou de carbonate de calcium semble améliorer l'activité biologique du revêtement.

Cependant les couches néoformées apparaissent tout au plus comme support, et l'adsorption de protéines est nécessaire pour permettre l'attachement des ostéoblastes et favoriser leur multiplication. Par conséquent, une surface extrêmement réactive avec une grande surface spécifique permet une grande capacité d'attachement des protéines et facilite l'adhésion et l'activité cellulaire.

On connaît classiquement plusieurs types de procédés pour réaliser des dépôts biocompatibles de phosphate de calcium à la surface des matériaux orthopédiques :
- des procédés à haute température, principalement par projection plasma,
- des procédés de recristallisation d'une phase apatitique à partir d'une phase gazeuse,
- des procédés de formation à partir d'une phase aqueuse généralement basés sur la nucléation et croissance à partir de solutions saturées du composé à déposer.

Actuellement, les dépôts d'hydroxyapatite ou de phosphate de calcium amorphe effectués par projection plasma sont couramment utilisés industriellement pour les implants denses, orthopédiques ou dentaires ; toutefois les dépôts obtenus par cette technique présentent une faible surface spécifique de l'ordre de 1 m²/g, donc une faible réactivité. D'autres procédés connus de recouvrement à haute température incluent l'immersion dans un sel fondu [Lacefield W.R. ; « Hydroxyapatite coatings », Bioceramics, Ducheyne P. and Lemons J.E. Eds., The New-York Acad. Sci., 72-80, 1988], la compression isostatique à chaud, la pulvérisation chimique [Frèche M., « Contribution à l'étude des phosphates de calcium, croissance sur le phosphate dicalcique anhydre, croissance des dépôts élaborés par pulvérisation chimique » ; Thèse d'Etat, INP Toulouse ; 1989].

D'autres méthodes telles que la pulvérisation ionique ou l'ablation laser ont également été proposées pour déposer une couche de phosphate de calcium sur divers substrats [Ong J.L., Lucas L.C., Lacefield W.R., Rigney E.D., « Structure, solubility and bond strength of thin calcium phosphate coatings produced by beam sputter deposition» ; Biomaterials, 13, 4, 249-254, 1992]. Ces méthodes basées sur le transfert des constituants minéraux en phase gazeuse sont relativement lentes et coûteuses et ne permettent pas de contrôler la composition de la phase minérale. Celle-ci est généralement amorphe lorsque le dépôt est effectué à basse température, et un traitement thermique simultané ou consécutif est nécessaire pour provoquer la formation d'une phase apatitique.

Des traitements en milieu aqueux sursaturés ont également été proposés pour favoriser la conversion d'une phase amorphe préalablement formée par des procédés physiques à sec, en apatite bien cristallisée (Lee D., Conner W. ; Hydroxyapatite coatings and a method of their manufacture, US patent 5,958,504, 1992).

Les recouvrements d'hydroxyapatite sont connus pour apporter un caractère au matériau à implanter, c'est à dire qu'il favorise la formation osseuse à son conctact (ostéoconduction) et facilite l'ancrage de la prothèse au tissu osseux (biointégration). L'ajout d'une couche de phosphate de calcium amorphe améliore considérablement la réactivité du matériau [Van Blitterswijk C.A., Bowell Y.P., Flash J.S., Leenders H., Van deer Brink I., De Bruijn ; Variation in hydroxyapatite cristallinity: effect on interface reactions ; Hydroxyapatite coatings in orthopaedic surgery,*ed R.G.T Geesink and M.T. Manley, Raven Press, New-York, 33-47, 1993] du fait de l'augmentation de la surface spécifique de celui-ci et de la grande solubilité des phases amorphes qui semble augmenter localement la sursaturation dans les fluides biologiques aux alentours de la surface de l'implant. Cependant, ces procèdès coûteux ne permettent pas de contrôler aisément la nature des phases formées, ni leur état de surface et leur réactivité, de plus les revêtements ne peuvent pas être associés à des principes actifs (facteurs de croissance, hormones, médicaments). Les phases obtenues sont en fin de compte assez éloignées des apatites biologiques.

C'est pour ces raisons que depuis quelques années de nombreuses recherches portent sur les dépôts effectués en solution à basse température. Les méthodes de recouvrement à basse température permettent d'obtenir une grande variété de structures et de composition des dépôts. En outre, certaines de ces méthodes permettent l'association de protéines ou de cellules actives au biomatériau.

La plupart de ces techniques mettent en oeuvre des solutions sursaturées, il s'agit alors de provoquer la précipitation de phosphate de calcium sur un substrat métallique. La manière la plus simple de procéder consiste à utiliser les propriétés de nucléation du métal plongé dans une solution sursaturée. Ainsi, après un temps de latence pendant lequel rien ne se produit, la cristallisation commence et la surface de l'échantillon se recouvre d'une couche de phosphate de calcium. Ces techniques ont toutefois l'inconvénient d'être lentes et de ne permettre de déposer que de petites quantités de phosphate de calcium, en raison des faibles concentrations des solutions sursaturées métastables. Aussi divers procédés dérivés ont été proposés dont certains ont fait l'objet de brevets. La précipitation peut être induite par le changement de sursaturation à proximité de la surface à traiter ; ainsi, T Kokubo et coll. [Kokubo T., Hata K., Nakamura T., Ymamuro T. ; «Apatite formation on ceramics metals and polymers induced by CaO-SiO₂ based glass in simulated body fluid » ; Bioceramics, 4, 113-120, 1991 et Kokubo T., Yamamuro T., Abe Y. ; Process for forming a bioactive hydroxyapatite film ; US patent 5,068,122 ; 1989] ont proposé une technique consistant à placer le substrat à proximité d'un verre à base de CaO. et SiO₂ dans des conditions de température, de pH, de concentration ionique, identiques à celles du corps humain. La libération d'ions calcium par le verre élève localement la sursaturation et conduit à la précipitation de phosphate de calcium sur le substrat. D'autres techniques utilisent l'électrolyse d'une solution sursaturée de phosphate de calcium. Lorsque le métal à traiter est employé comme cathode, l'électrolyse provoque une libération locale d'ions hydroxydes à la surface du métal. Cette élévation du pH conduit à l'augmentation de la sursaturation et provoque la précipitation du phosphate de calcium sur le substrat [P. Royer, « Etude du recouvrement à basse température de matériaux orthopédiques par des phosphates de calcium », Thèse INPT, 1992]. On peut également citer la méthode d'électrodéposition sur des alliages de titane [Ducheyne P., Van Raemdonck W., Heughebaert J.C., Heughebaert M. ; « Calcium phosphate ceramic coatings on porous titanium : effect of the structure and composition on electrophoretic déposition, vacuum sintering and in vitro dissolution » ; Biomaterials, 11, 244-254, 1990].

Les procédés basés sur l'utilisation de solutions sursaturées ne permettent cependant pas de déposer des quantités importantes de phosphate de calcium en raison de l'instabilité des solutions et de la précipitation spontanée de phosphate de calcium si la concentration en sel dépasse quelques millimoles par litre. Afin de contourner cette difficulté Layrolle (Layrolle P, de Groot K., de Bruijn J., van Blitterswijk C., Huipin Y. « Method for coating médical implants » EP 98203085, 1998) a proposé d'utiliser des solutions 4 à 5 fois plus concentrées que celles habituellement utilisées mais stabilisées par des pH plus acides obtenus par dissolution de gaz acides (dioxyde de carbone). Le dégagement de gaz produit une élévation du pH et la précipitation du phosphate -de calcium. Les revêtements ainsi obtenus restent cependant long à former (plusieurs heures) et font au mieux quelques microns d'épaisseur.

Des procédés utilisant la croissance cristalline à composition constante [Nancollas G. H., Purdie N., « The kinetics of crystal growth », Quart. Rev. Chem. Soc., 18, 1-20, 1964] permettent un contrôle de la composition chimique du dépôt puisque la consommation des ions Ca²⁺ et des groupements phosphates dans le réacteur est constamment compensée à l'aide de burettes automatiques connectées au pH-mètre. Ce procédé ne peut être cependant utilisé que pour traiter des implants de petite surface. Il est ainsi difficilement transposable au niveau industriel.

Toutes les techniques de précipitation à partir de solutions sursaturées ne permettent pas d'obtenir des dépôts d'épaisseur appréciable. Celle-ci est en général limitée à quelques micromètres, précisément parce qu'il n'est pas possible de travailler avec des solutions concentrées. Les durées de formation de ces dépôts sont relativement longues, quelques heures le plus souvent et les minéraux déposés peuvent maturer et perdre une grande partie de leur réactivité potentielle. Le dépôt s'effectue dans toute la cuve, ce qui diminue le rendement (la partie uniquement déposée sur la prothèse) et nécessite un nettoyage. Enfin les solutions sursaturées sont d'un emploi délicat à l'échelle industrielle puisqu'elles sont instables. Les poussières, les défauts sur les surfaces sont autant de sites qui peuvent permettre à une précipitation parasite de se produire.

Toutes ces techniques permettent toutefois d'obtenir des couches de phosphate de calcium mal cristallisées, non-stoechiométriques et relativement proches du minéral osseux. Cependant, certaines de ces méthodes conduisent à une composition du dépôt hétérogène due à la modification de la composition de la solution au cours du traitement. De plus, la phase déposée évolue au cours du traitement ce qui induit une diminution de la réactivité.

De façon connue, ces procédés présentent donc tous l'inconvénient d'une mise en oeuvre difficile compte tenu de l'instabilité et de la réactivité des couches déposées qui limitent leur utilisation au stade industriel.

L'invention vise un procédé de mise en oeuvre simple, rapide et à moindre coût, permettant de résoudre les problèmes pré-cités.

L'invention propose à cet effet un procédé permettant de recouvrir une surface, éventuellement poreuse, d'un substrat -notamment un implant ou une prothèse chirurgical orthopédique- par une couche de phosphate de calcium apatique. Un procédé conforme à la présente invention est mis en oeuvre à basse température -notamment dans une plage de température de l'ordre de 0 à 100°C- et se caractérise en ce qu'on utilise une suspension aqueuse de phosphate amorphe adaptée pour induire une germination et une croissance cristallines de la phase apatitique. Cette phase de phosphate amorphe est plus soluble que la phase apatitique et lorsque les germes cristallins d'apatite se forment à la surface du substrat leur croissance est constamment alimentée par la dissolution de la phase amorphe qui rétablit la sursaturation de la solution. Un procédé selon l'invention ne nécessite pas l'utilisation de solutions sursaturées, ce qui facilite son développement industriel. Il permet en outre une formation rapide de revêtements d'épaisseur contrôlée.

Avantageusement, un procédé selon l'invention comprend les étapes suivantes :
a) on prépare une suspension fraîche de phosphate amorphe,
b) on applique cette suspension sur la surface à revêtir -notamment par pulvérisation, immersion du substrat dans la suspension (dip-coating), simplement à l'aide d'une brosse-,
c) on initie la germination et la croissance cristallines du phosphate apatitique sur le substrat.

Avantageusement et selon l'invention, on contrôle la germination et la croissance cristallines par différents moyens -notamment par une germination naturelle sur des substrats adaptés, par une variation de la température, par une variation du pH dans une plage de l'ordre de 4,5-12, par une variation de la concentration locale en calcium ou en phosphate, dans une plage de concentration de l'ordre de 0 à 1 M-. Cette disposition qui permet de s'affranchir de l'utilisation d'appareillages coûteux, de (utilisation délicate de solutions sursaturées, tout en restant comparativement relativement rapide, conduit à un coût de fabrication réduit.

Avantageusement et selon l'invention, le contrôle de la germination et de la croissance cristallines peut également se réaliser en utilisant des inhibiteurs de la germination et/ou de la croissance cristallines. Selon cet aspect de l'invention, on utilise avantageusement une concentration inférieure ou égale à 1 M d'au moins un inhibiteur choisi parmi un des ions suivants : l'ion magnésium, l'ion carbonate, l'ion pyrophosphate.

Avantageusement et selon l'invention, le contrôle de la germination et de la croissance cristallines de la phase apatique est réalisé par des variations simultanées ou consécutives d'au moins deux des paramètres précédemment exposés.

La description d'un mode préféré de réalisation de l'invention, donné ci-après, permettra de mieux comprendre les buts et avantages de l'invention. Il est clair que cette description est donnée à titre d'exemple, et n'a pas de caractère limitatif.

Le phosphate amorphe est précipité d'après la méthode décrite par Heughebaert (Heughebaert J.C. ; Contribution à l'évolution des orthophosphates de calcium précipités amorphes en orthophosphates apatitiques, Thèse ENSCT, 1977) ou ses variantes. Elle consiste en une double décomposition entre une solution de sel de phosphate soluble (pouvant éventuellement contenir des ions carbonate ou d'autres ions) et de sel de calcium soluble (pouvant éventuellement contenir d'autres ions). Le précipité obtenu est lavé et récupéré par filtration. Il est ensuite mis en suspension dans une solution aqueuse de manière à obtenir une pâte fluide contenant entre 80 et 98 % d'eau.

Cette suspension est appliquée par divers moyens (imprégnation, brosse, pulvérisation) sur la surface à revêtir. Le revêtement à la brosse est la technique la plus facile pour les surfaces non poreuses. Elle permet de délimiter précisément les zones à revêtir, mais elle ne permet pas d'obtenir une épaisseur constante et reproductible.

La pulvérisation est également facile à mettre en oeuvre pour des surfaces non-poreuses. Elle peut être répétée et permet un contrôle fin de l'épaisseur du dépôt.

L'imprégnation est la technique préférée pour les surfaces poreuses. La densité de la suspension aqueuse de phosphate de calcium amorphe permet de contrôler l'épaisseur du dépôt..

On peut faciliter la pénétration de la suspension dans les pores par la mise sous vide partiel (10 à 30 mm de Hg typiquement) qui facilite le départ de l'air occlus et son remplacement par la suspension. Cette procédure peut être répétée.

La germination cristalline et la croissance de la phase apatitique sur certains substrats peuvent se produire naturellement lorsque ceux-ci possèdent la faculté de faciliter sa formation (substrats à base d'apatite, de titane ou de ses oxydes, d'oxyde de silicium etc,...). La formation de la phase apatitique peut être modifiée et facilitée sur ces substrats ou être initiée sur d'autres par différents moyens :
- variation de la température choisie dans le domaine entre 0 et 100°C. Il est connu en effet que les phosphates de calcium ont une solubilité qui varie en fonction de la température. Par conséquent la sursaturation relative des suspensions peut être modifiée par la température. Typiquement une température élevée facilite la formation de la phase apatitique à partir de la suspension mais elle ne permet pas d'obtenir des phases réactives mal cristallisées. Ces dernières se forment préférentiellement dans le domaine 0-40°C. On pent avantageusement choisir la température physiologique (37°C) qui permet une germination et une croissance cristallines rapides tout en préservant la grande réactivité de la phase formée.
- variation de pH choisi dans le domaine 4,5-12. L'élévation du pH facilite la formation d'apatites stoechiométriques, mais stabilise également (au-dessus de pH 10) la phase amorphe. Le pH physiologique (7,4) semble le plus approprié pour la formation d'apatites biomimétiques.
- variation de la concentration en calcium et phosphate (0 à 1 M). L'élévation de la concentration en calcium et/ou phosphate permet de modifier la germination de la phase apatitique et sa composition. La germination est en général facilitée par une élévation de la concentration en calcium et/ou phosphate. La présence de ces ions dans la solution permet en outre de modifier la stoechiométrie de la phase apatitique : des solutions riches en calcium facilitent, par exemple, la formation d'apatites plus proches de la stoechiométrie.
- présence d'inhibiteurs tels que les ions carbonate, magnésium ou pyrophosphate (0 à 1 M). Ces ions inhibent la croissance de la phase apatitique et stabilisent également la phase amorphe. Ils permettent cependant de contrôler la taille des cristallites formées. Ces ions sont également des inhibiteurs de la maturation de la phase apatitique et ils permettent de préserver sa grande réactivité. Dans le but d'obtenir des apatites biomimétiques la conversion de la phase amorphe pourra être effectuée en présence de ces ions dans la solution, notamment les ions carbonate et magnésium qui modifient la composition de surface de la phase apatitique...).

Un procédé selon l'invention comporte donc une précipitation d'un précurseur amorphe puis sa conversion en apatite qui s'effectue directement à la surface de l'implant ce qui permet l'obtention d'une phase très immature et par conséquent très réactive. Le principal avantage de ce procédé est sa simplicité et son faible coût par rapport aux procédés existants.

Il est connu que les apatites mal cristallisées caractérisées par une grande surface spécifique possèdent des propriétés d'adsorption considérables leur conférant ainsi une grande réactivité. La réactivité de cette phase favorise l'adsorption d'éléments (notamment bioactifs) mais favorise aussi les interactions avec la matière organique, ce qui a pour but d'améliorer considérablement « l'ostéoconduction ». L'amélioration des propriétés ostéoconductives des implants permet l'utilisation de ce procédé dans de nombreuses applications, en particulier lors d'implantation sur des sujets âgés ou des sujets présentant des os endommagés ou affaiblis.

Les caractéristiques d'un procédé selon l'invention permettent de déposer des minéraux biocompatibles éventuellement associés à des molécules actives plus rapidement et à un coût plus faible.

Un tel procédé est par ailleurs beaucoup plus aisé à mettre en oeuvre puisqu'il n'utilise pas de solutions sursaturées qui sont d'emploi délicat à l'échelle industrielle. L'épaisseur des dépôts de matériaux biocompatibles est directement liée à la densité de la suspension aqueuse de phosphate de calcium amorphe et à ses propriétés de mouillabilité des surfaces, elle ne peut avoir lieu que sur la prothèse, d'où un moindre coût de mise en oeuvre.

La présente invention permet donc de déposer, à basse température, sur des surfaces quelconques et notamment des surfaces d'implants chirurgicaux poreux ou denses une phase minérale biocompatible, adhérente, d'épaisseur variable, résorbable ou non-résorbable selon sa composition, constituée de phosphate de calcium apatitique.

Elle s'applique en particulier au domaine du revêtement de prothèses orthopédiques métalliques par des matériaux biocompatibles qui facilitent leur biointégration.

De nombreuses variantes peuvent être considérées en fonction des circonstances d'utilisation, tant en ce qui concerne la nature des matériaux traités, généralement à base de titane ou d'alliage chrome-cobalt, qu'en ce qui concerne la nature des revêtements réalisés.

Il est également possible de réaliser des dépôts multi-couche par répétition du procédé en utilisant des conditions d'hydrolyse différentes. La répétition du traitement permet l'obtention de dépôts d'épaisseurs très différentes, mais permet aussi de superposer des dépôts de phases différentes : hydroxyapatite stoechiométrique à la surface du matériau à recouvrir ce qui permet d'obtenir une phase non-résorbable biocompatible, puis une ou plusieurs phases très réactives possédant une grande surface spécifique de l'ordre de 200 m²/g ce qui favorise l'adsorption d'éléments bioactifs (notamment des ions Mg²⁺, Sr²⁺, F⁻, Mn²⁺, VO₄³⁻) et facilite les interactions minéral-organique.

Par ailleurs, un procédé selon l'invention permet d'associer par adsorption des molécules biologiquement actives au dépôt, favorisant la réparation tissulaire (protéines osseuses spécifiques telles que : ostéocalcine, ostéonectine, ostéopontine, etc., facteurs de croissance) ou de principes actifs (agents antimicrobiens, antibiotiques). Le revêtement constitue un vecteur intéressant de principes actifs puisqu'il est en contact direct avec l'os et qu'il peut se résorber progressivement, selon sa composition, pour être finalement remplacé par une partie d'os nouvellement formé.

Un autre avantage de la méthode réside dans la possibilité d'incorporer à la couche apatitique mal cristallisée soit au moment de sa formation soit après sa formation (après l'étape de germination et de croissance cristalline) des ions minéraux bioactifs tels les ions magnésium (Mg²⁺), strontium (Sr²⁺), fluorures (F⁻), manganèse (Mn²⁺), vanadate (VO₄³⁻)... (Marie et *al.,* J. Bone Miner. Res. 8 : 607-615, 1993). Ces ions peuvent soit faire partie de la phase amorphe de départ, à savoir la solution aqueuse de la suspension de phosphate de calcium amorphe, soit être introduits par un simple échange dans la couche d'apatite très réactive après sa formation (Cazalbou S. ; thèse INPT, Toulouse, 2000). Cette dernière solution permet de fixer les ions à la surface des cristaux et d'accroître leur activité biologique.

Enfin la couche déposée, potentiellement biomimétique, permet l'adhésion de cellules impliquées dans la reconstruction osseuse telles les cellules ostéoblastiques et ostéoclastiques ou les cellules souches précurseurs de ces cellules.

D'autres caractéristiques de l'invention apparaîtront à la lecture des exemples suivants donnés à titre illustratif et non limitatif. Les figures 1 à 4 illustrent les résultats obtenus dans les exemples.

### EXEMPLE 1 :

La phase de phosphate amorphe est obtenue par double décomposition entre une solution de calcium et une solution de phosphate, identifié par son spectre Infrarouge (figure 1).

La solution de calcium est obtenue par dissolution de 46,3 g de Ca(NO₃)₂, 4 H₂O dans 500 ml d'eau auquel on ajoute 50 ml de NH₄OH.

La solution de phosphate est obtenue par dissolution de 27,2 g de (NH₄)₂HPO₄ dans 1300 ml d'eau auquel on ajoute 50 ml de NH₄OH.

La solution de calcium est ensuite versée dans la solution de phosphate puis filtrée et lavée abondamment avec de l'eau désionisée.

Le gel ainsi obtenu est mis à nouveau en suspension (27 g de gel dans 100 ml d'eau désionisée).

Une plaque de titane à recouvrir de dimensions 10 cm par 3 cm est placée dans la suspension précédemment obtenue. La plaque ainsi recouverte est placée dans une solution d'eau à 60°C pendant 3 heures de manière à effectuer l'hydrolyse de la phase amorphe en phosphate de calcium apatitique.

### EXEMPLE 2 :

La phase de phosphate amorphe est obtenue par double décomposition entre une solution de calcium et une solution de phosphate.

La solution de calcium est obtenue par dissolution de 46,3 g de Ca(NO₃)₂, 4 H₂O dans 500 ml d'eau auquel on ajoute 50 ml de NH₄OH.

La solution de phosphate est obtenue par dissolution de 27,2 g de (NH₄)₂HPO₄ dans 1300 ml d'eau auquel on ajoute 50 ml de NH₄OH.

La solution de calcium est ensuite versée dans la solution de phosphate puis lavée abondamment avec de l'eau désionisée. Le gel ainsi obtenu est mis à nouveau en suspension (27 g de gel dans 100 ml d'eau désionisée).

La suspension précédemment obtenue est appliquée par pulvérisation sur une plaque de titane de dimension 10 cm par 3 cm.

La plaque ainsi recouverte est placée dans une solution d'eau à 25°C pendant 15 heures de manière à effectuer l'hydrolyse de la phase amorphe en phosphate de calcium apatitique. La phase apatitique peut-être identifiée par son spectre Infrarouge (Figure 3).

### EXEMPLE 3 :

La phase de phosphate amorphe est obtenue par double décomposition entre une solution de calcium et une solution de phosphate.

La solution de calcium est obtenue par dissolution de 46,3 g de Ca(NO₃)₂, 4 H₂O dans 500 ml d'eau auquel on ajoute 50 ml de NH₄OH.

La solution de phosphate est obtenue par dissolution de 27,2 g de (NH₄)₂HPO₄ dans 1300 ml d'eau auquel on ajoute 50 ml de NH₄OH.

La solution de calcium est ensuite versée dans la solution de phosphate puis lavée abondamment avec de l'eau désionisée. Le gel ainsi obtenu est mis à nouveau en suspension (27 g de gel dans 100 ml d'eau désionisée).

Une plaque de titane à recouvrir de dimensions 10 cm par 3 cm est placée dans la suspension précédemment obtenue. La plaque ainsi recouverte est placée dans une solution contenant 2 g Ca(NO₃)₂ et 0,3 ml NH₄OH à 30 % pour 200 ml d'eau à 60°C pçndant 30 minutes de manière à effectuer l'hydrolyse de la phase amorphe en phosphate de calcium apatitique. La figure 2 montre le spectre Infrarouge du dépôt obtenu. II s'agit d'une apatite mal cristallisée pauvre en ions hydroxyde.

### EXEMPLE 4 :

La phase de phosphate amorphe est obtenue par double décomposition entre une solution de calcium et une solution de phosphate.

La solution de calcium est obtenue par dissolution de 46,3 g de Ca(NO₃)₂, 4 H₂O dans 500 ml d'eau auquel on ajoute 50 ml de NH₄OH.

La solution de phosphate est obtenue par dissolution de 27,2 g de (NH₄)₂HPO₄ dans 1300 ml d'eau auquel on ajoute 50 ml de NH₄OH.

La solution de calcium est ensuite versée dans la solution de phosphate puis lavée abondamment avec de l'eau désionisée. Le gel ainsi obtenu est mis à nouveau en suspension (27 g de gel dans 100 ml d'eau désionisée).

La suspension précédemment obtenue est appliquée à l'aide d'un pinceau sur une plaque de dimension 10 cm sur 3 cm.

La plaque ainsi recouverte est placée dans une solution contenant 2 g NaF pour 200 ml d'eau à 60°C pendant 30 minutes de manière à

## Revendications

1. Procédé permettant de recouvrir une surface, éventuellement poreuse, d'un substrat par une couche de phosphate de calcium apatitique nanocristallin, ledit procédé étant mis en oeuvre à basse température -notamment dans une plage de température de l'ordre de 0 à 100°C- et étant **caractérisé en ce qu'**on utilise une suspension aqueuse de phosphate amorphe adaptée pour induire une germination et une croissance cristallines de la phase apatitique.

2. Procédé selon la revendication 1, **caractérisée en ce qu'**elle comprend les étapes suivantes :
a) on prépare une suspension aqueuse fraîche de phosphate amorphe,
b) on applique cette suspension sur la surface du substrat à revêtir -notamment par pulvérisation, immersion du substrat dans la suspension, application à l'aide d'une brosse-,
c) on initie la germination et la croissance cristallines.

3. Procédé selon la revendication 2, **caractérisé en ce qu'**on contrôle la germination et la croissance cristallines de la phase apatitique sur la surface du substrat.

4. Procédé selon les revendications 1 ou 2, **caractérisé en ce que** la densité de la suspension aqueuse de phosphate amorphe est choisie selon l'épaisseur désirée de la couche de phosphate de calcium.

5. Procédé selon les revendications 3 ou 4, **caractérisé en ce que** le contrôle de la germination et de la croissance cristallines de la phase apatitique est réalisé par des variations de la température -notamment dans une plage de l'ordre de 0 à 100°C-.

6. Procédé selon la revendication 5, **caractérisé en ce que** le contrôle de la germination et de la croissance cristallines de la phase apatitique est réalisé par des variations de la température -notamment dans une plage de l'ordre de 0 à 40°C-.

7. Procédé selon les revendications 3 ou 4, **caractérisé en ce que** le contrôle de la germination et de la croissance cristallines de la phase apatitique est réalisé par des variations du pH -notamment dans un domaine de l'ordre de 4,5 à 12-.

8. Procédé selon les revendications 3 ou 4, **caractérisé en ce que** le contrôle de la germination et de la croissance cristallines de la phase apatitique est réalisé par des variations de la concentration locale en calcium et/ou phosphate -notamment dans un domaine de l'ordre de 0 à 1 M-.

9. Procédé selon les revendications 3 ou 4, **caractérisé en ce que** le contrôle de la germination et de la croissance cristallines de la phase apatitique est réalisé en utilisant des inhibiteurs de la germination et/ou de la croissance cristalline.

10. Procédé selon la revendication 9, **caractérisé en ce que** les inhibiteurs de la germination et/ou de la croissance cristallines sont utilisés à une concentration inférieure ou égale à 1 M, et sont choisis parmi au moins un des ions suivants : l'ion magnésium, l'ion carbonate, l'ion pyrophosphate.

11. Procédé selon les revendications 3 ou 4, **caractérisé en ce que** la germination et la croissance cristallines sont contrôlées par des variations simultanées ou consécutives d'au moins deux paramètres choisis parmi : la température, le pH, la concentration en calcium, la concentration en phosphate, la présence d'inhibiteurs de germination et/ou de croissance cristalline(s).

12. Procédé selon l'une des revendications 1 à 11, **caractérisé en ce qu'**on effectue des dépôts multi-couches en répétant les différentes étapes dans les mêmes conditions ou dans des conditions différentes.

13. Procédé selon l'une des revendications 1 à 12, **caractérisé en ce que** la germination et la croissance cristallines sont effectuées en présence de molécules bioactives -notamment des antibiotiques, des facteurs de croissance, des protéines osseuses spécifiques (ostéocalcine, ostéonectine, ostéopontine,...)-.

14. Procédé selon la revendication 13, **caractérisé en ce que** les molécules bioactives sont fixées par adsorption sur les cristallites après l'étape de germination et de croissance cristallines.

15. Procédé selon la revendication 14, **caractérisé en ce que** les molécules bioactives sont fixées par adsorption ou par échange ionique sur les cristallites après l'étape de germination et de croissance cristallines.

16. Procédé selon l'une dès revendications 1 à 15, **caractérisé en ce que** la phase de phosphate amorphe de la suspension contient des éléments biologiques actifs -notamment des ions Mg²⁺, Sr²⁺, F⁻, Mn²⁺, VO₄³⁻.

17. Procédé selon la revendication 16, **caractérisé en ce que** la solution aqueuse de la suspension contient les éléments bioactifs.

18. Procédé selon l'une des revendications 1 à 17, **caractérisé en ce que** les surfaces traitées sont associées à des cellules impliquées dans la reconstruction des tissus biologiques -notamment des cellules souches, des ostéoblastes-.
